# EUROPEAN PATENT SPECIFICATION

(11) **EP 1 223 940 B1**
(45) Date of publication and mention of the grant of the patent: **27.12.2006**
(21) Application number: 00969591.7
(22) Date of filing: 13.10.2000
(51) Int. Cl.: A61K 31/565, A61P 19/10, A61P 15/12, A61P 5/30

(54) **USE OF AN ESTROGENIC COMPOUND FOR THE TREATMENT OF OSTEOPOROSIS**
DIE VERWENDUNG VON ESTROGEN-VERBINDUNGEN ZUR BEHANDLUNG VON OSTEOPOROSE
L'UTILISATION DES OESTROGENES POUR LE TRAITEMENT DE L'OSTEOPOROSE

(30) Priority: 15.10.1999 US 159501 P
(43) Date of publication of application: 24.07.2002
(73) Proprietor: Orion Corporation, 02200 Espoo (FI)
(72) Inventor: TIMONEN, Ulla, FIN-02400 Kirkkonummi (FI); VAHERI, Raija, FIN-00330 Helsinki (FI)
(74) Representative: Vossius & Partner
(86) International application number: PCT/FI2000/000890
(87) International publication number: WO 2001/026640

(56) References cited:
- EP-A- 0 393 539
- EP-A- 0 461 290
- WO-A-95/22332
- HEIKKINEN J E ET AL: "Optimizing continuous-combined hormone replacement therapy for postmenopausal women: a comparison of six different treatment regimens" AM J OBSTET GYNECOL, vol. 182, no. 3, March 2000 (2000-03), pages 560-567, XP002901505
- HEIKKINEN J ET AL: "Low-dose continuous combined HRT increases BMD in postmenopausal women" MATURITAS, vol. 35, no. 1, June 2000 (2000-06), pages s66-s67, XP002901506
- HEIKKINEN J ET AL: "Effects of long-term continuous combined HRT on endometrial histology" MATURITAS, vol. 35, no. 1, June 2000 (2000-06), page s67 XP002901507
- TIMONEN U ET AL: "Comparison of three continuous combined estrogen-progestin regimens" MATURITAS, vol. 35, no. 1, June 2000 (2000-06), page s67 XP002901508

## Description

### FIELD OF THE INVENTION

The present invention relates to the use of an estrogenic compound in the manufacture of a medicament for the prevention or treatment of postmenopausal osteoporosis characterized in that the treatment is initiated with a low dose of an estrogenic compound and after the initiation period the dose of the estrogenic compound is increased, doubled.

### BACKGROUND OF THE INVENTION

Osteoporosis is a systemic skeletal disease characterized by low bone mass and microarchitectural deterioration of bone tissue, with a consequent increase in bone fragility and susceptibility to fracture. It is a significant cause of mortality and morbidity in the elderly people, both of individual and community perspective. Osteoporosis affects an estimeted 75 million people in Europe, the United States and Japan.

The number of postmenopausal women has been increasing steadily with the ageing population. One of the most common types of osteoporosis is associated with menopause. Annual losses of 2-6% of bone mass have been reported to occur in women in early postmenopause.

Conventional estrogen preparations are currently used for the treatment of postmenopausal osteoporosis. One such preparation is a sequentially administered preparation containing 2 mg of estradiol valerate combined with 10 mg of medroxyprogesterone acetate (Divina®, Orion Corporation, Espoo, Finland).

### SUMMARY OF THE INVENTION

The invention relates to the use of an estrogenic compound in the manufacture of a medicament for the prevention or treatment of postmenopausal osteoporosis characterized in that the treatment is initiated with a low dose of an estrogenic compound and after the initiation period the dose of the estrogenic compound is doubled.

The initiation period of the treatment usually lasts about 4 to 8 months, preferably 6 months.

### BRIEF DESCRIPTION OF THE DARWINGS

Figure 1 shows the change (%) in bone mineral density in osteopenic and osteoprotic women in lumbar spine.
Figure 2 shows the change (%) in bone mineral density in osteopenic and osteoprotic women in femoral neck.

### DETAILED DESCRIPTION OF THE INVENTION

The inventors suprisingly discovered that when treating postmenopausal osteoporosis by initiating the treatment with a low dose of an estrogenic compound and increasing the dose of the estrogenic compound after this initiation period, the increase in bone mineral density was higher than when treating postmenopausal osteoporosis by administering the same dose of the estrogenic compound (either the low dose or the increased dose) for the whole term of the treatment. Particularly, it was found that in patients whose daily dose of the estrogenic compound was increased after the first 6 months of the treatment from 1 mg of estradiol valerate (E₂V) to 2mg of E₂V, the increase in bone mineral density in the vertebrae, expressed as a percentage, was 8.9 % after the treatment term of 48 months. Analogously, the increase in bone mineral density, expressed as a percentage, was 6.2 % and 7.4 % in patients whose daily estrogen dose during the whole treatment term was either 1 mg of E₂V or 2 mg of E₂V respectively. The corresponding values in the femoral neck were 4.2 %, 2.9 % and 2.9 %. Further, it was found that in osteopenic and osteoporotic patients whose daily dose of the estrogenic compound was increased after the first 6 months of the treatment from 1 mg of E₂V to 2mg of E₂V, the increase in bone mineral density in the vertebrae, expressed as a percentage, was 9.4 % after the treatment term of 48 months. Analogously, the increase in bone mineral density, expressed as a percentage, was 7.7 % and 7.3 % in patients whose daily estrogen dose during the whole treatment term was either 1 mg of E₂V or 2 mg of E₂V, respectively. In osteopenic and osteoporotic patients the corresponding values in the femoral neck were 6.4 %, 3.8 % and 3.5 %.

The present invention relates to the prevention and treatment of postmenopausal osteoporosis which comprises initiating the treatment with a low dose of an estrogenic compound and after this initiation period doubling the dose of the estrogenic compound. The initiation period of the treatment usually lasts about 4 to 8 months, preferably 6 months.

The low dose of an estrogenic compound used during the initiation period is a dose equivalent in estrogenic activity to approximately 0.5 mg - 1.5 mg of estradiol valerate. Preferably, the low dose of an estrogenic compound is a dose equivalent in estrogenic activity to approximately 1 mg of estradiol valerate.

The doubled dose of an estrogenic compound used during the rest of the treatment term is a dose equivalent in estrogenic activity to 1 mg - 3 mg of estradiol valerate. Preferably, the increased dose of an estrogenic compound is a dose equivalent in estrogenic activity to 2 mg of estradiol valerate.

The estrogenic compound of the present invention is preferably estradiol valerate. Other conventional estrogens such as estrone, estrone sulfate, estrone sulphate piperazine salts and their esthers as well as synthetic estrogens may also be employed.

### EXAMPLE

In order to demonstrate the effectiveness of using the method of the present invention, and its advantages, a comparative study was undertaken to evaluate the efficacy and tolarability of the method. The subjects used in the study were 419 postmenopausal women, aged 45 to 65 years. Eligible women had to be at least 3 years postmenopausal with normal gynecological findings, an intact uterus and a body mass index of 30 or less. Major medical exclusion criteria included endometrial hyperplasia and pathological mammography findings, prior hormone-dependent malignancies, severe thyroid diseases, and other criteria typical for HRT studies. Excessive smokers and alcohol abusers were excluded. Otherwise the women were not required to change their daily diet or other lifestyle habits for the study period. The women did not take any calcium or vitamin D supplementation during the study period.

Subjects were randomly assigned to six treatment groups to receive one tablet daily containing one of the following dose combinations: 1 mg or 2 mg of estradiolvalerate (E₂V) combined to 2.5 mg or 5 mg of medroxyprogestrone acetate (MPA) (Indivina®, Orion Corporation, Espoo, Finland).

| Group | Daily E₂V dose | Daily MPA dose |
|---|---|---|
| | (First 6 mo/After 6 mo) | |
| | | |
| Group I | 1 mg / 2 mg | 2.5 mg |
| Group II | 1 mg/2 mg | 5 mg |
| | | |
| Group III | 2 mg /2 mg | 2.5 mg |
| Group IV | 2 mg / 2 mg | 5 mg |
| | | |
| Group V | 1 mg / 1 mg | 2.5 mg |
| Group VI | 1 mg / 1 mg | 5 mg |

Groups changing from 1 mg E₂V to 2 mg E₂V irrespective of the MPA (Group I and II) were pooled together. Groups receiving 1 mg of E₂V throughout the study irrespective of the MPA dose (Group V and VI) were pooled together. Also groups receiving 2 mg of E₂V throughout the study irrespective of the MPA dose (Group III and IV) were pooled together. Pooling was considered justified since MPA is not expected to affect the response of estrogen on bone mineral density (BMD).

The bone mineral densities at the vertebrae and the hip were measured using dual-energy X-ray absorptiometry (DPX) at baseline, 6-, 12-, 24-, 36- and 48-month follow-up visits, and at premature discontinuation. The BMD from the hip was measured by three separate measurements at the femur neck, Ward's triangle, major trochanter and BMD from the vertebrae was measured at L2-L4. In addition, the area and bone mineral content (BMC) of vertebrae L2-L4 were determined.

### Bone mineral density at 48 months

Both vertebral and femoral bone mineral densities increased in all three groups. The increases were more pronounced at the vertebrae, but significant improvement was also observed at all indices at the hip (femoral neck, trochanter and Ward's triangle).

### Pooled results of all subjects

In the vertebrae there was a statistically significant difference in favor of the 2 mg estrogen dose. The mean percentage change was 7.4% in the group receiving 2 mg of E₂V compared to 6.2% in the group receiving 1 mg of E₂V (p=0.0079). Similarly, BMD response in group switching from 1 mg to 2 mg E₂V differed statistically significantly from 1 mg E₂V group 8.9%, (p<0.001).

In the femoral neck no significant difference could be seen between lower and higher estrogen dose groups, but the group switching from 1 mg to 2 mg had statistically significantly larger BMD response compared to 1 mg of E₂V group (4.3% vs. 2.9%, p=0.0361).

### Osteopenic/osteoporotic vs normal bones in pooled groups

Results were further analysed to compare the effect varying dosages on BMD in groups that had either normal, osteopenic or osteoporotic BMD value at baseline. Subjects were classified as osteopenic if the BMD was 0.90 tol .08 g/cm² in vertebrae (L2-L4) and 0.68 to 0.86 g/cm² in femoral neck (corresponds to T score between -1 and - 2.5 in Finnish population). BMD was regarded as normal if the values were above 1.08 g/cm² in vertebrae and above 0.86 g/cm² in femoral neck. Values below 0.90 g/cm² in vertebrae and below 0.68 g/cm² in femoral neck were considered as osteoporotic.

In vertebrae, the mean change from baseline in osteopenic/osteoporotic subjects (BMD T score below -1) was 7.3% in 2 mg of E₂V group, 7.7% in 1 mg E₂V and 9.4% in 1 mg/2 mg E₂V group, no significant differences were seen. For the group with normal BMD, corresponding changes were 7.5%, 4.7% and 8.3%. Both the 1 mg/2 mg E₂V group and 2 mg of E₂V group differed statistically significantly from the 1 mg E₂V group (p<0.001).

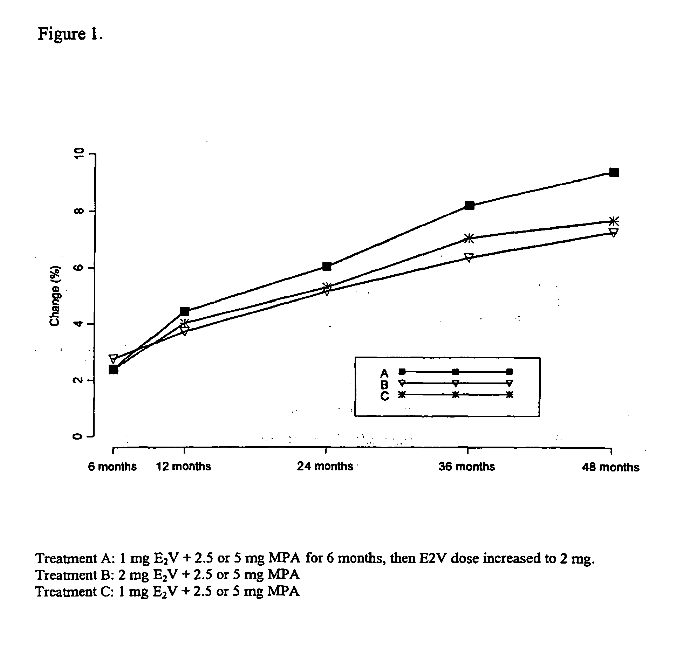

Vertebral BMD and change (%) in BMD from baseline can be seen in Figure 1.

In femoral neck, the BMD changes ranged from 2.1% to 2.5% in the group that had normal BMD values at baseline. For osteopenic/osteoporotic subjects, the percentage changes were 6.4% for the 1 mg/2 mg E₂V group, 3.5% for 2 mg E₂V group and 3.8% for 1 mg of E₂V group. The differences between 1 mg/ 2 mg of E₂V group and the two other groups were statistically significant (1 mg/ 2 mg vs. 2 mg p=0.0258 and 1 mg /2 mg vs. 1 mg p=0.0086).

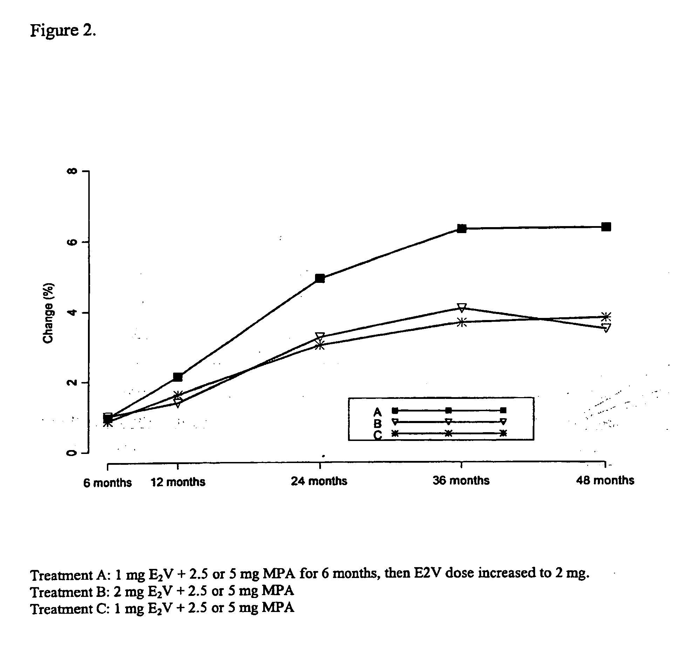

Femoral BMD and change (%) in BMD from baseline can be seen in Figure 2.

The most prominent increase for all incides measured was seen in women who had first been taken 1 mg E₂V for 6 months before switching over to the 2 mg regimen. The baseline bone mineral density values were somewhat, but not significantly, lower in this group, which may explain the obvious superiority of this group in gaining bone mineral density after six months. However, there were no differences in baseline bone mineral density of women with low bone mass between the groups, and nevertheless greater vertebral and particularly femoral bone mineral density increases were seen in these women. Furthermore, about half of women in each group had used some kind of HRT previously. Thus, there was no unbalanced distribution of previous HRT users in the three groups which could have been resulted in slower bone mineral density gain in group with a higher number of previous users. Morever, there were no other differences in the baseline characteristics between the groups that could have affected the results.

## Claims

1. Use of an estrogenic compound in the manufacture of a medicament for the prevention or treatment of postmenopausal osteoporosis **characterized by** a daily dose of an estrogenic compound equivalent in estrogenic activity to 0.5 to 1.5 mg of estradiol valerate during an initiation period lasting from 4 to 8 months and an increased, doubled daily dose of said estrogenic compound after the initiation period.

2. The use according to claim 1, wherein the initiation period lasts 6 months.

3. The use according to any one of claims 1 to 2, wherein the daily dose of the estrogenic compound in the initiation period is a dose equivalent in estrogenic activity to 1mg of estradiol valerate.

4. The use according to any one of claim 1 to 3, wherein the initiation period is 6 months and the daily dose is 1 mg of estradiol valerate and the daily dose of estradiol valerate after said initiation period is 2 mg.

## Patentansprüche

1. Verwendung einer Östrogen-Verbindung für die Herstellung eines Arzneimittels zur Prävention oder Behandlung von Postmenopausen-Osteoporose, **gekennzeichnet durch** eine tägliche Dosis einer Östrogen-Verbindung, die einer Östrogen-Aktivität von 0,5 bis 1,5 mg Estradiolvalerat entspricht, während einer Anfangsperiode, welche 4 bis 8 Monate dauert, und eine gesteigerte, doppelte tägliche Dosis der Östrogen-Verbindung nach der Anfangsperiode.

2. Verwendung nach Anspruch 1, wobei die Anfangsperiode 6 Monate dauert.

3. Verwendung nach einem der Ansprüche 1 bis 2, wobei die tägliche Dosis der Östrogen-Verbindung während der Anfangsperiode eine Dosis ist, die der Östrogen-Aktivität von 1 mg Estradiolvalerat entspricht.

4. Verwendung nach einem der Ansprüche 1 bis 3, wobei die Anfangsperiode 6 Monate beträgt und die tägliche Dosis 1 mg Estradiolvalerat ist und die tägliche Dosis Estradiolvalerat nach der Anfangsperiode 2 mg ist.

## Revendications

1. Utilisation d'un composé oestrogénique pour la fabrication d'un médicament pour la prévention ou le traitement de l'ostéoporose post-ménopausique, **caractérisée par** une dose quotidienne d'un composé oestrogénique équivalent, en activité oestrogénique, à 0,5 à 1,5 mg de valérate d'oestradiol pendant la période d'initiation durant de 4 à 8 mois et une dose quotidienne augmentée, doublée, dudit composé oestrogénique après la période d'initiation.

2. Utilisation selon la revendication 1, dans laquelle la période d'initiation dure 6 mois.

3. Utilisation selon l'une quelconque des revendications 1 à 2, dans laquelle la dose quotidienne du composé oestrogénique dans la période d'initiation est une dose équivalente, en activité oestrogénique, à 1 mg de valérate d'oestradiol.

4. Utilisation selon l'une quelconque des revendications 1 à 3, dans laquelle la période d'initiation est de 6 mois, et la dose quotidienne est de 1 mg de valérate d'oestradiol, et la dose quotidienne de valérate d'oestradiol après ladite période d'initiation est de 2 mg.
